# EUROPEAN PATENT SPECIFICATION

(11) **EP 2 641 083 B1**
(45) Date of publication and mention of the grant of the patent: **10.04.2019**
(21) Application number: 11836806.7
(22) Date of filing: 14.09.2011
(51) Int. Cl.: G01N 1/38, G01N 33/24

(54) **MICROSAMPLING NUTRIENT MEASUREMENT**
MIKROPROBEN-NÄHRSTOFFMESSUNG
ANALYSE DE NUTRIMENTS PAR MICRO-ÉCHANTILLONNAGE

(30) Priority: 29.10.2010 US 455993 P; 13.09.2011 US 201113231701; 22.05.2011 US 201161488765 P
(43) Date of publication of application: 25.09.2013
(73) Proprietor: Solum, Inc., Mountain View, CA 94043 (US)
(72) Inventor: KOSHNICK, Nicholas, Carleton, Mountain View, CA 94043 (US); PREINER, Michael, John, Mountain View, CA 94043 (US); WHITE, Justin, Stewart, Mountain View, CA 94043 (US)
(74) Representative: Boult Wade Tennant LLP
(86) International application number: PCT/US2011/051625
(87) International publication number: WO 2012/057929

(56) References cited:
- EP-A1- 1 203 955
- WO-A1-2010/107121
- JP-A- 2005 331 409
- US-A- 5 136 249
- US-A- 5 526 705
- US-A- 5 850 620
- US-A1- 2003 110 871
- US-A1- 2004 248 283
- US-A1- 2006 088 939
- US-A1- 2007 073 491
- US-A1- 2010 283 993
- US-B1- 6 937 939

## Description

### FIELD OF THE INVENTION

The present invention relates generally to soil test preparation and soil measurement, and more particularly to measurement of soil properties for example for precision agriculture.

### BACKGROUND OF THE INVENTION

Farmers often make soil nutrient management decisions based on field sample test results. These field samples are often collected by agronomists or service providers and then sent to soil chemistry labs for the analysis. The most common attribute measurements (phosphorus, potassium, soil pH, etc.) use a standardized process where the sample is first air or oven dried for some period of time, and then ground up. The dried, ground up soil is then treated with an extractant and is measured in this state. This drying and grinding process insures that the (approximately 2 gram) subsample that is used for each chemical analysis adequately represents the much larger field sample (approximately 0.23 to 0.45kg (0.5 to 1 pound)) that is sent to the lab. Elimination of the drying and grinding process would significantly reduce the labor and mailing costs for the chemical and physical analysis of soils.

The drying and grinding process is also slow. The typical process involves a day or more of drying the samples and, therefore, measurements cannot be delivered in real-time. Laboratories often compete with each other on the response time that they can deliver from the time the samples are delivered to the lab to the time test results are available. Elimination of the drying and grinding process would significantly reduce the turn-around time for the chemical and physical analysis of soils.

Further, the drying and grinding practice is also inaccurate. Drying changes the mineralogy of the soil samples, affecting the measurement of soil nutrients. Finally, existing soil measurements systems must dedicate additional time and labor to separating and cataloguing each field sample that arrives at the analysis site, resulting in errors that must be corrected. This also adds to the overall cost and turn-around time.

In US5,526,705, an apparatus analyzes a plurality of soil samples to determine characteristics of the soil samples. An input portion sequentially provides a plurality of soil samples each having a known solid content. A plurality of vessels are supported for movement with a continuous conveyor relative to the input portion to receive the soil samples. A plurality of testing stations are arranged relative to the continuous conveyor to sequentially access the samples carried by the vessels. The testing stations each test the samples to determine at least one of the characteristics of the samples.

### BRIEF SUMMARY

A process for homogenizing and measuring soil-water samples provides for rapid, robust analysis of large numbers of field samples in a short time frame. This removes the need for dry-and-grind type testing.

The invention is disclosed in the independent claim 1. Additional embodiments are disclosed in the dependent claims.

Time and cost savings associated with using the process allow for area-based pricing and time-based pricing of soil analysis, possibly independent of the density at which field samples are tested.

### BRIEF DESCRIPTION OF THE DRAWINGS

FIG. 1 illustrates a process for homogenizing and measuring soil-water samples, in accordance with one embodiment.
FIG. 2 illustrates a process for homogenizing and measuring attributes of soil-water samples at two locations remote from one another, in accordance with one embodiment.
FIG. 3 illustrates a soil-water device for homogenizing and measuring soil-water subsamples, and for preparing soil-water subsamples to be transported in a test well device.
FIG. 4A illustrates a test well device.
FIG. 4B illustrates a double layer seal for a test well device.
FIG. 5a illustrates a side view of a test well device configured to add multiple extractants to soil-water subsamples.
FIG. 5b illustrates a top view of a test well device configured to add multiple extractants to soil-water subsamples.

The figures depict embodiments of the present invention for purposes of illustration only. One skilled in the art will readily recognize from the following discussion that alternative embodiments of the methods illustrated herein may be employed without departing from the principles of the invention described herein.

### DETAILED DESCRIPTION OF PREFERRED EMBODIMENTS

### Field Sample Measurement

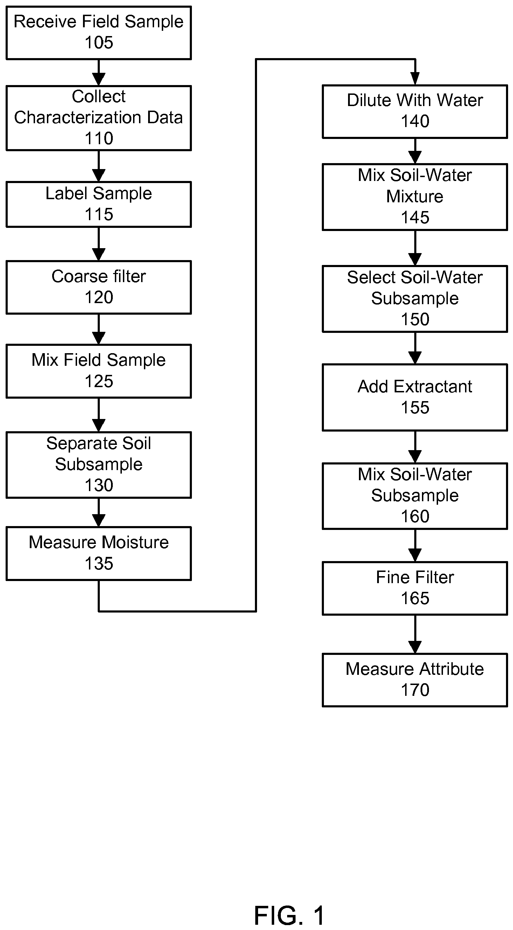

FIG. 1 illustrates a process for homogenizing and measuring soil-water samples, in accordance with one embodiment. A field sample of soil is obtained from the ground, and is transported to the location where the field sample is going to be measured. The field sample may be of variable size. The field sample should be large enough such that the amount taken is representative of the local area of the field to be measured, but small enough so that it is convenient to process and transport the field sample. For example, a 100 gram or a one kilogram sample are both large enough to be representative of the area of the field to be measured, but small enough to process and transport. Field samples may be collected manually, or they may be collected with an automated field soil collection device. The automated field soil collection device may, for example, be attached to a farming instrument such as a tractor, ATV, truck, or a handheld unit.

The field sample is received 105 at the location where the field sample is to be measured. Field samples may be measured anywhere there is a device to perform the desired measurements. This may include measurement using a device capable of operating in the field itself, at a local field office near the field, or at a laboratory located remotely from the field or field office, including laboratories that are, for example, more than 400 miles, or an overnight driving distance or flying distance away.

Characterization data is collected 110 regarding the field sample. Characterization provides additional information regarding the field sample that is relevant but cannot be obtained by testing the field sample. Characterization data includes, for example, a location where the field sample was obtained, a sample owner or other agent who is responsible for the field sample, a time the field sample was obtained, a date the field sample was obtained, and who obtained the field sample.

With respect to the location where the field sample was taken, the location may include a place that is georeferenced by a global navigation satellite system such as a global positional system (GPS), by a map, or by any other locating system. Location information may also include metadata regarding generalized location information. For example, even though ten field samples may have individual GPS locations associated with them, they might have all been pulled from the same field or planting, and thus may be associated with a single field location according to the field owner or the testing laboratory.

Characterization data may also be collected 110 throughout the entire process. Thus, characterization data may also include the amount of time the soil-water mixture or soil-water subsample was mixed, weather information when the field sample was taken (e.g., temperature, humidity, and barometric pressure), sample collection information (e.g., a sample weight, a picture of the sample core, and a picture of a composite sample), and sample moisture information (e.g., using time domain reflectance, capacitive soil moisture, inductive moisture, expressed soil vapor, or gravimetric readouts while air drying or heating the sample).

The field sample is labeled 115 so that it is uniquely identified and may be tracked throughout the measurement process. The label stays with the field sample throughout processing and measurement. Thus, the label provided to a field sample applies even after it is converted to a soil-water mixture or transferred into a soil-water sample. If a single field sample is split into multiple soil-water subsamples, a sub-label can be provided to each subsample. The label may take the form of an RFID or bar code, so that the label may be easily read by a computer. By labeling samples, results of a measurement of a field sample may be automatically communicated to the sample owner. The label may vary in what information it contains. The label may contain, for example, any piece of characterization data, or the results obtained from testing a sample once they are known.

A field sample may be passed through a coarse filter 120 in order to remove objects that are not part of the soil, such as rocks or other foreign objects. Coarse filtering 120 may consist of pressing the field sample through a 0.635cm (quarter-inch) screen, though other size screens may be used. Water may also be added to the field sample to speed up the coarse filtering 120 so that the soil passes through the filter more readily.

The field sample is mixed 125 to increase its homogeneity. A soil subsample is then separated 130 from the field sample as a whole, where the soil subsample will be the portion the field sample that is used in one or more measurements. The soil subsample may also consist of the entire field sample, minus small amounts of field sample that are lost during handling. The mixing 125 of the field sample ensures that the soil subsample is representative of the field sample as a whole. To facilitate the mixing 125 and separation 130 of the soil subsample, water may be added to the field sample. Mixing 125 may be performed by stirring, shaking, sonication, or any other mechanism.

In one embodiment, multiple subsamples may be taken as the field sample (or subsequent soil-water subsamples) are mixed in order to test the effect of mixing time length on measurement of soil properties. Information regarding mixing time length may be used to calibrate the amount of time samples or subsamples are mixed for.

The moisture of the soil subsample is measured 135 in order to determine how much water to add prior to measurement. Frequently, obtained field samples will be field-moist, meaning that they already have some amount of water in them. Coarse filtering 120 and mixing 125 may also have added some water to the soil.

There are a number of different techniques that may be used to measure moisture. If the soil subsample has been allowed to dry, a change in weight of the soil subsample due to water lost through evaporation can be used to determine the moisture present in the sample, or to reduce the amount of moisture to zero before more water is added. Moisture may also be measured by measuring the capacitance or conductivity of the soil subsample, as a soil subsample with relatively more water will have a higher conductivity and a lower capacitance. Moisture may further be measured in the slurry.

Moisture may also be measured optically, by using a light source in the 1000 to 200 nm wavelength range to measure the reflectance or attenuation of the light with respect to the soil subsample. For example, dips in the reflectance spectrum centered around 1400 and 1850 nm may be used to determined the moisture content of the soil. The features at these wavelengths can be compared to reference measurements, to a standardized database of measurements, or to measurements of the reflectance after different amounts of drying of the soil subsample to determine the moisture content of the soil subsample.

Based on the amount of moisture in the soil subsample, the soil subsample is diluted with water 140 to form a soil-water mixture. The water used throughout this process may be regular tap water, or deionized water. In some cases, flocculent salts may also be added to the soil-water mixture to facilitate fine filtering of the soil-water subsample that may occur later in the process.

The soil subsample is diluted 140 until a dilution ratio is achieved. The dilution ratio is chosen so as to provide the best measurement of the attribute of interest. For example, in one case the dilution ratio may be anywhere between one part water to two parts soil, to thirty-five parts water to one part soil, inclusive, by weight or by volume. Typically, the dilution ratio will be between one part water to two parts soil, to five parts water to one part soil, inclusive, by weight or by volume.

In examples falling outside the scope of the invention, dilution 140 may also be accomplished by diluting a field sample with water by one of a few fixed amounts, independent of the exact weight or content of the soil subsample. In this case, the dilution 140 is not performed to reach a specific dilution ratio, but rather simply to create a soil-water mixture that can later be portioned and precisely tuned to reach a specific dilution ratio. For example, dilution 140 may include adding 500 mL of water if the soil subsample was taken from 15.24cm (six inch) cores of field sample, 750 mL of water if the soil subsample was taken from 20.32cm (eight inch) cores of field sample, or one liter of water if the soil subsample was taken from 30.48cm (twelve inch) cores of field sample. As different users have different standards for the depth of the core they take when they obtain a field sample, tailoring the dilution 140 to expected core sizes add convenience to the dilution 140 part of the process.

The soil-water mixture is mixed 145 to increase its homogeneity. A soil-water subsample is selected 150 from the soil-water mixture, where the soil-water subsample will be the portion of the soil- water mixture that is used in measurement. The mixing 145 of the soil-water mixture ensures that the soil-water subsample is representative of the soil-water mixture as a whole. Mixing 145 may be performed by stirring, shaking, sonication, or any other mechanism.

Depending upon the measurement to be performed, additional extractants may be added 155 to the soil-water subsample. Water itself may be considered an extractant. In some cases, additional water is added to the soil-water subsample in order to reach a dilution ratio. This may be the case, for example, if the earlier dilution 140 of the soil subsample to form the soil-water mixture was not performed to achieve a specific dilution ratio. The ranges of possible dilution ratios for the soil-water subsample may be the same as the ranges of possible dilution ratios for the soil-water mixture listed above.

Prior to measurement, the soil-water subsample is mixed 160. In part, mixing serves to stir up soil that might have settled out. In some cases, the soil-water subsample is also fine filtered 165. Fine filtering filters particulates of a smaller granularity than coarse filtering 120. An example of a fine filter includes filter paper, such as coffee filter paper, though more sophisticated filters may be used as well. Filtering is particularly useful for measurements where the attribute to be measured is the quantity of a substance that is highly water soluble, for example nitrate-nitrogen. If the substance to be measured is water soluble, the mixing processes 160 and 145 will have caused the substance to be separated from the soil. Thus, the fine filtering removes soil particulate that may interfere with measurement. Another example of fine filtering 15 includes centrifuging the soil-water subsample in a centrifuge. Centrifuging causes particles within the soil-water subsample to separate from each other based on density. Filtering and centrifuging also stop the extraction process, as particulates in the soil subsample have been filtered or centrifuged out. After filtering and/or centrifuging, portions of the soil-water subsample may be transferred for measurement 170.

The soil-water subsample is then measured 170. An attribute of the original field sample is determined by analyzing the results provided by the measurement 170. The results of the measurement may be reported to the sample owner using the information contained in the label. The concentration of nitrate-nitrogen in the field sample is an example of a measurement of an attribute that may be performed. The process described in FIG.1 may be repeated multiple times, once for each attribute to be measured. In some cases, some steps can be skipped for subsequent attribute measurements, as a single field sample, soil subsample, soil-water mixture, or soil-water subsample may hold up to repeated measurements 170.

### Local and Remote Field Sample Attribute Measurement

In some cases, it is preferable to perform a first attribute measurement in a location near to (or in) the field where the field sample was obtained, for example at a local field office, and to perform other measurements at a laboratory located remotely from the field or any local field office. There are numerous reasons why this might be the case, for example because some measurements are time sensitive and must be performed quickly whereas others can wait, or because in-field measurements are costly compared to their laboratory counterparts.

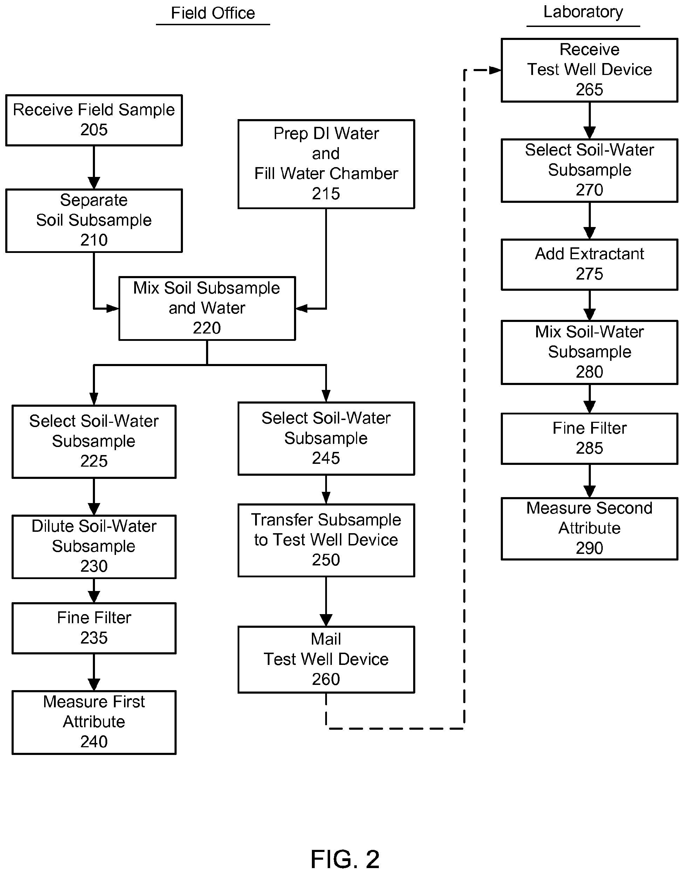

FIG. 2 illustrates a process for homogenizing and measuring attributes of soil-water samples at two locations remote from one another, in accordance with one embodiment. In the example process of FIG. 2, a first measurement is performed near (or in) the field where the field sample was obtained. Additionally, a set of soil-water subsamples are prepared and stored in a test well device, which is mailed for testing at a remote location, such as a laboratory. Many steps of the process described in FIG. 2 carry over from FIG.1. Thus, some steps mentioned in FIG. 1 may be added to the process of FIG. 2.

A field sample is received 205 for testing. The field sample may be received at a location in the field or at a field office. A soil subsample is separated 210 from the field sample, after mixing the field sample to ensure that the soil subsample is representative of the field sample. The soil subsample may also consist of the entire field sample, minus small amounts of field sample that are lost during handling. Deionized water may be prepared and transferred into a water chamber 215. As the preparation of deionized water is time consuming, this preparation may occur concurrently with the receiving 205 and separating 210 steps. In cases where regular tap water is used in place deionized water, the time needed to prepare and fill the water chamber 215 will be minimal.

The water from the fill chamber and the soil subsample are mixed 220 to form a soil-water mixture. As above, mixing ensures that soil-water subsamples are representative of the contents of the soil-water mixture. The soil-water mixture is used for two purposes, both of which may be carried out concurrently.

First, a first soil-water subsample is selected 225 to be used in a measurement to be performed proximally to the location where the field sample was taken. The soil-water subsample is diluted 230. Additional extractants may be added to the soil-water subsample to facilitate measurement. Salts may be added to the soil-water subsample to facilitate fine filtering. The soil-water subsample may be filtered 235. A first attribute is measured 240 using the first soil-water subsample. The results of the measurement may be immediately reported to the sample owner, in part due to the close proximity between where the field sample was taken and where the measurement was performed. The concentration of nitrate-nitrogen in the field sample is an example of a first measurement of a first attribute that may be performed on the first soil-water subsample close to the location where the field sample was obtained.

Second, a second soil-water subsample is selected 245 to be stored and mailed for later measurement. The second soil-water subsample is transferred to a test well device comprising a number of test wells. A label may also be attached to the test well containing the second soil-water subsample. The selection 245 of soil-water subsamples may be repeated many times for a given soil-water mixture, soil subsample, or field sample. The soil-water subsamples are transferred to the to the test well device 250.

The first and second soil-water subsamples need not be identical. In a first example, the second soil-water subsample may be similar to the first soil-water subsample. Both subsamples may be relatively precise and accurate volume where each subsample includes a specific volume of soil and water needed for measurement. In a second example, the second soil-water subsample may be of a relatively large volume compared to the first soil-water subsample. In this case, the second soil-water subsample is large enough so that it may be split up at the remote location for use in multiple measurements. A larger volume is beneficial if the tests at the remote location require different volumes for testing, and it is not known in advance which tests will be performed. Further, this allows for greater flexibility in the design of the test well device.

In one design, the test well device comprises extractant wells in addition to test wells. Extractants in the extractant wells may be added to the test wells before or after the soil-water subsamples have been added to the test wells. By adding extractants to the test wells, extraction can occur before or during transit, thereby saving time in the measurement process once the test well device arrives at the remote location for testing. Adding extractant during transit may also make the extracted solutions more stable, mitigating the degradation of soil nutrients during transit. The addition of extractants to the test wells may also be followed by mixing and filtering, where filtering would cause extraction to cease.

The test well device is mailed or otherwise transported 260 to a remote location so that the included subsamples may be measured. The test well device containing the soil-water subsamples is received 265 at the remote location. For testing, an individual soil-water subsample is transferred 270 from a well in the test well device. In the case of the first example above, the entire volume is used if the volumes of the soil-water subsamples were precisely set in advance. In the case of the second example, a portion of the entire volume of the test well is selected 270 and transferred out of the test well device.

The soil-water subsample is mixed 280, either before or after transferring the soil-water subsample out of the test well device. Depending upon the measurement to be performed, extractants are added 275 and mixed in to the soil-water subsample, if the nutrients have not already been extracted. The soil-water subsample may also be passed through a fine filter 285. The soil-water subsample is then measured 290. A second attribute of the original field sample is determined by analyzing the results provided by the measurement 290. The results of the measurement may be reported to the sample owner using the information contained on the associated label. The process may be repeated for each of the soil-water subsamples in the test well device.

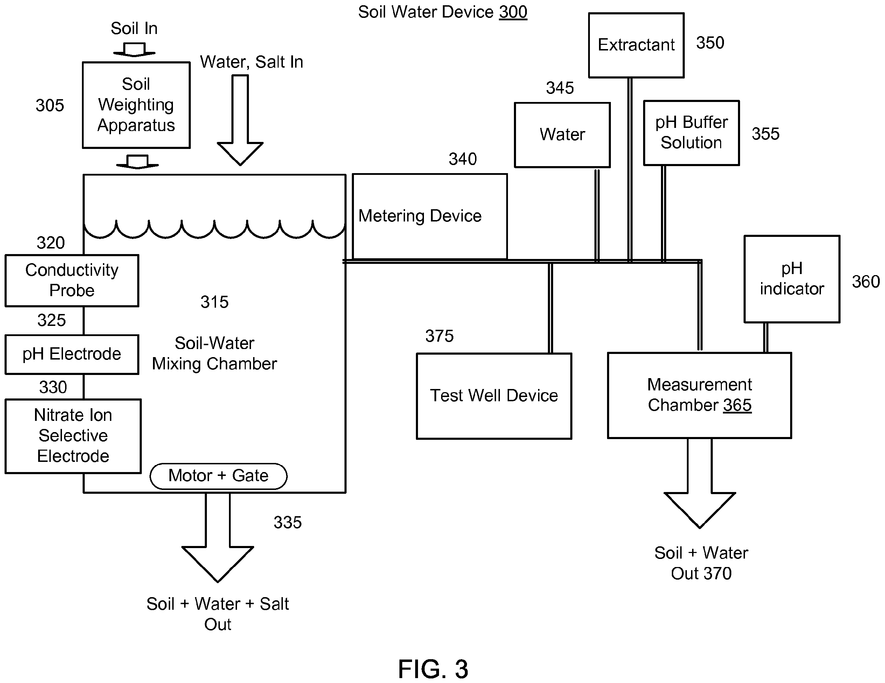

The subsamples of the test well device that are measured 290 remotely may be measured using the same type of device that performs the measurement of the first attribute. The measurements of both processes 170, 240, and 290 may be performed using the device described in FIG. 3. The measurements 290 may also be performed using inductively coupled plasma atomic emission spectroscopy (ICP-AES), using a flame photometer, using a flow injection analyzer, and/or using a pH electrode, among other devices. One purpose of transporting the test well device 375 to a laboratory from the field or field office is to gain access to more powerful methods of instrumentation, so there is a great deal of flexibility in how the soil-water subsamples of the test well device are processed and measured.

### Soil Water Device

FIG. 3 illustrates a soil-water device for homogenizing and measuring soil-water samples, and for preparing soil-water samples to be transported in a test well device. The soil-water device 300 includes a soil-water mixing chamber 315, a metering device 340 for selecting and transferring soil-water subsamples, a measurement chamber 365 for measuring attributes of a soil-water subsample. The soil-water device 300 can also accommodate a test well device 375 to store soil-water subsamples for later testing. The soil-water device 300 is designed to hold liquids, and thus the soil-water device 300 also includes barriers (e.g., valves) and transmission mechanisms (e.g., tubes) for transporting liquids between the components of the device 300.

Soil and water are received and mixed into a soil-water mixture in the soil-mixing chamber 315. The soil-water mixing chamber 315 includes an opening to receive water and soil. The water that is mixed with the soil could be regular tap water, deionized water, or some other water that has undergone some form of treatment. In some cases, the soil added to the soil-water mixing chamber is added directly after being scooped from another source. In other cases, the soil is weighed using a soil weighing apparatus 305 prior to being added to the soil-water mixing chamber 315. The soil weighting apparatus may also be used to determine the moisture present in the soil. The moisture content of the soil is used to determine how much water to add to the soil-water mixing chamber 315.

The soil-water mixing chamber 315 may mix its contents using different mechanisms. Examples of techniques for mixing include using a motor attached to a set of blades (e.g., a blender), via sonication, stirring, or shaking. In the case of a blender, a turbulator may be added to decrease the time needed to mix the contents of the chamber. The bottom of the soil-water mixing chamber 315 may include a gate 335 for emptying the contents of the chamber.

The soil-water mixing chamber 315 may also include electronic probes to obtain additional information about the field sample. Examples of probes include a conductivity probe 320, a pH electrode 325, and ion selective electrodes 330 including membranes for various nutrients such as nitrate and potassium. The measurements performed by the electronic probes may be used to supplement measurements performed with the measurement chamber 365. For example, pH may be measured using a pH electrode 325, as well as by adding a pH buffer solution 355 to the measurement chamber 355 and using a pH indicator 360 to measure pH.

One or more metering devices 340 are coupled to the soil-water mixing chamber 315, and are used to select soil-water subsamples and to transfer them from the mixing chamber to the test well device 375 and the measurement chamber 365. Depending upon the construction of the soil water device 300, the same metering device 340 may be used to select and transfer soil-water subsamples for both the test well device 375 and the measurement chamber 365. In other constructions, separate metering devices 340 may be separately used to select and transfer soil-water subsamples for the test well device 375 and the measurement chamber 365. Separate metering devices 340 may be useful if the volume of the soil-water subsample to be transferred into the measurement chamber 365 is significantly different in volume from the volume of the soil-water subsamples transferred into the test well device 375. Separate metering devices 340 may also be useful if the soil-water subsample to be transferred into the measurement chamber 365 is of a precise and accurate volume as compared to the soil-water subsamples transferred into the test well device 375. The metering device 340 may be, for example, a graduated pipette.

The soil-water subsample transferred from the soil-water mixing chamber 315 can contain a sizable portion of air if it was being very vigorously mixed when it was transferred. The metering device 340 allows for a stage where the air can settle out in the extracted soil-water subsample without reducing the mixing speed in the soil-water mixing chamber 315. The metering device 340 also allows the volume of the soil-water subsample to be read out manually (e.g., by visual examination) or in an automated (e.g., optical or electronic) manner.

The measurement chamber 365 receives a soil-water subsample, and performs a measurement on the subsample. The measurement chamber 365 may include an optical measurement chamber for performing spectroscopic measurements. An optical measurement chamber may be used to perform many different nutrient measurements of a field sample including, for example, nitrate-nitrogen, phosphorus, potassium, zinc, sulfur, ammoniacal nitrogen, and amino acid nitrogen. The measurement chamber 365 may also measure field sample properties, such as particulate size, pH, conductivity, organic matter content, mineralogy, and texture. For organic matter, broad band spectroscopy can be used to determine organic matter content. For soil texture, the percentage of sand, silt, and clay present in a sample can be determined. As with organic matter, broad band spectroscopy can be used, in either transmission, side-scattering, or reflectance modes. For nitrate - nitrogen measurement, the UV-visible range absorption spectroscopy can be used.

In addition to measuring soils, an optical measurement chamber may measure plant tissue (e.g., leaves, stalks, petioles, roots), as well as water (such as field drainage water) containing biological material. For biological material, the measurement chamber 365 may measure pesticide levels, biological activity levels (e.g., total fungus levels and bacterial levels), and specific biological organisms (e.g., nematodes and salmonella) directly or after amplification by growing the organisms in cultures.

The soil to water ratio in the soil-water subsample may be between 2:1-1:5, inclusive, and in some cases between 2:1-1:35, inclusive, or any other ratio that is convenient or preferable for a particular measurement. The measurement chamber 365 may also receive additional water 345, extractants 350, and pH buffer solution 355 to perform additional measurements or to further refine the contents of the soil-water subsample. The measurement chamber 365 may also be connected to a pH indicator, for example phenol red.

In some cases, the measurement chamber 365 may include a fine filter (not shown) for filtering the soil-water sample prior to measurement. Example of fine filters include paper filters (e.g., a coffee filter), a centrifuge, and a reusable filter (e.g., a porous stainless steel filter), however other filters may be used. Salts used in conjunction with filtering may introduced directly in the measurement chamber 365, or may be added in the soil-water mixing chamber 315. In one embodiment, the added salt is a flocculent that binds to particulates in the soil-water sample, thereby increasing the efficiency and efficacy of filtration.

In one case, a soil-water mixing chamber 315 and an optical measurement chamber 365 may be used as described in intermediate publication US2010/0283993.

The measurement chamber 365 may include an outlet port 370 for emptying the contents of the measurement chamber 365. In order to mix the contents added to the measurement chamber to the soil-water subsample, the measurement chamber 365 may also include mixing mechanism (not shown).

The soil water device 300 may also include a labeling device (not shown) for tracking and transmitting information related to the field sample and its related soil-water subsamples. A computer system (not shown) may be associated with the soil water device 300 for transmitting sample related information as labeled samples pass through different parts of the measurement process. For example, progress can be reported once the field sample is separated, mixed with water, separated into subsamples, once the test well device is prepared, and during and after measurement of subsamples. The computer system may automatically transmit label information to the sample owner, to the field office where the samples are initially processed, and to the laboratory where the samples in the test well device 375 are processed.

The soil water device 300 may be mounted on a vehicle such as an all-terrain vehicle, a tractor, a pickup truck, or trailer. This way, field samples may be input into the device 300 at the site where the field samples were taken. The soil water device 300 may also include a device for determining its location, for example via a global positioning system, so that the location where the field sample was taken may be determined automatically when the sample is loaded into the soil water device 300.

In one example, the soil water device 300 can be used to reproduce a traditional "Bray and Kurtz" type measurement. One run of the Bray and Kurtz method involves weighing 2 grams of air-dried or field moist soil, adding 20 mL of Bray and Kurtz extracting solution, shaking for 5 minutes, and filtering the sample immediately. The extractant solution was prepared by adding 4.12 mL of concentrated hydrochloric acid to approximately 1.8 liters of DI water, and then adding 2.22 grams of NH₄F. After mixing, this mixture is brought to 2L with DI water, and the pH is adjusted to 2.6 with HCl or NH₄OH. The soil water device 300 may also be used to reproduce a traditional Mehlich 3 type measurement.

One process for achieving equivalent results with the soil water device 300 is as follows: Weigh 60 grams of air dried or field moist soil, add the soil and 300 mL of deionized water together to the measurement chamber 315, and mix for two minutes. Transfer a soil-water subsample of 10 mL into the measurement chamber 365. Add 10 mL of a modified 2x extractant 350. Mix for 5 minutes, and then filter immediately. The modified 2x extractant 350 is produced in a similar manner by mixing 8.24 mL concentrated hydrochloric acid to approximately 1.8L DI water, then adding 4.44 grams NH₄F and mixing thoroughly, bringing the volume to 2 liters with DI water, and adjusting the pH to 2.3 with HCl or NH₄OH. The measurement in the measurement chamber 365 is performed according to the Murphy Riley phosphorus method.

The soil water device 300 is capable of reproducing the Illinois sidedress nitrate test, which traditionally involves adding sodium hydroxide directly to dry soil. In this case, the sodium hydroxide may be added to a soil-water subsample some time after it was originally mixed. Boric acid may added to a separate chamber of the measurement chamber 365 to capture out gassed ammonia through a gas outlet port (not shown) of the measurement chamber 365 for later titration and determination of the amino sugar nitrogen and inorganic nitrogen in the sample.

### Test Well Device

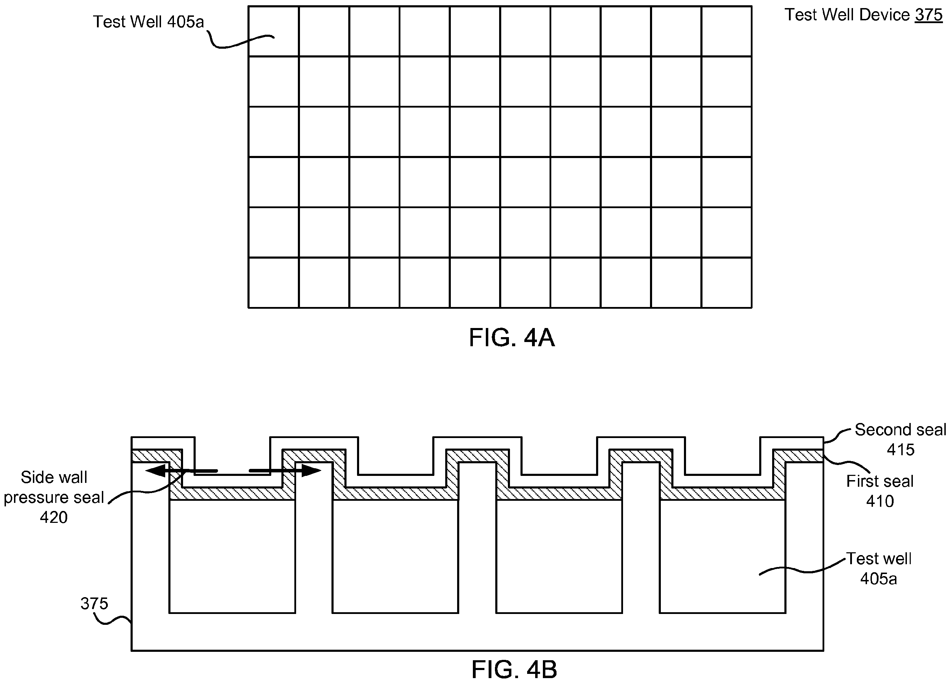

FIG. 4 illustrates a test well device. Test well device 375 includes a number of test wells 405, each of which holds a volume of liquid. The volume of each test well 405 holds a soil-water subsample. The soil-water subsample, however, may be of a variable volume, and therefore the size of the test wells may vary between test well devices. Some soil-water subsamples are small, and are entirely used up in a single measurement or run of measurements. Other soil-water subsamples are larger, such that during remote processing they may split for use in multiple measurements or runs of measurements. In some cases, test wells within a given test well device 375 may vary in size. The test well device may have only a single large test well, or it may have upwards of thousands of test wells. Typically, the test well device 375 will have between 24 and 96 wells. In one example, each test well has a volume of 24 mL.

FIG. 4A illustrates one example layout for the test well device 375, with identical test wells 405 with rectangular shape. In other examples, the test wells 405 are circular or oval shaped. Other geometries for the layout of the test well device 375 are possible. For example, the test well device may be a circular carousel of test wells.

FIG. 4B illustrates one example of a double layer seal for the test well device 375, to ensure that the contents of the test wells 405 do not leak during transit. The double layer seal includes a first layer 410 which covers the test well device 375. The first layer 410 is recessed into each test well 405. The first layer 410 may, for example, be constructed of rubber, plastic, or other materials. The second layer 415, covers the first layer 415, and recesses into the first layer 410 in each test well 405. The second layer 415 exerts horizontal pressure 420 on the first layer 410, which in turn exterts horizontal pressure 420 on the side wall of the test wells 405. This prevents fluid from leaking out of the test wells 405 by travelling between the boundary between the first layer 410 and the test well 405 side wall.

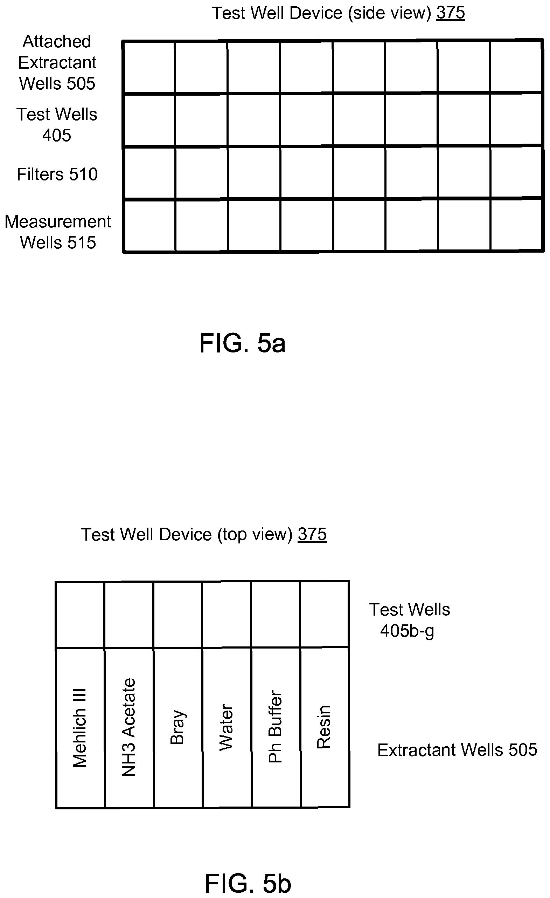

FIG. 5a illustrates a side view of a test well device configured to add multiple extractants to soil-water subsamples. In some cases, it is advantageous to prepare soil-water subsamples contained in the test well device 375 prior to their arrival at a remote laboratory for testing. To prepare the soil-water subsamples, the test well device 375 may include additional wells not containing soil-water subsamples.

In one case, the test well device 375 includes extractant wells 505 that are attachable to the top of the test well device 375. Each extractant well 505 contains an extractant. At some point between when the soil-water subsamples are loaded into the test well device 375, and when they are unloaded, the wells that contain the extractants 505 are attached to the top of the test wells 405 to add the extractant to the test wells 405.

FIG. 5b illustrates a top view of a test well device 375 configured to add multiple extractants to soil-water subsamples. In the example of FIG. 5b, soil-water wells 405b-g are all assumed to have a soil-water sample originating from the same field sample. Extractant wells 505 are built into the test well device 375, and are located on the top of the test well device. Each extractant well 505 includes a different extractant, for example Mehlich I, II, or III, Ammonium Acetate, Bray extract to measure phosphorus, Olsen extractant, Morgan extractant, additional water, pH buffer solution, and resins such as bicarbonate and chloride form anion exchange resins that can be used to measure various soil nutrients. In one example, Extractants are added to the test wells 405 by removing a seal between the extractant wells 505 and the test wells 405. Alternatively, the extractant wells 505 may require coupling to the test wells, wherein once the wells are coupled, inverting the extractant wells 505 causes the extractant to enter the test wells 405 (or vice versa). In another example, extractants from the extractants well 505 may be added by manual or automatic pipetting. Once the extractants have been added to the associated test wells 405b-g, the extractants and soil-water subsamples may be mixed. Mixing may be accomplished by shaking, stirring, sonication, or other mechanisms. Mixing parameters (temperature, speed, acceleration, etc) may be recorded during this process, and associated with the subsamples via the labels associated with the samples.

The soil-water subsamples in test wells 405may further be filtered to stop further reactions with any added extractants, stopping biological activity and thereby stabilizing the soil-water subsamples. Filtering stops the extraction process by removing biological material which would otherwise further react with the extractants. The test well device 375 may include filters 510 for this purpose. Filtering is initiated by exposing the test wells 405 to the filters 510, which may occur by removing a cover at the base of the test wells 405 on top of the filters 510. The soil-water subsample then passes through the filter 510 into a measurement well 515, where it sits until unloaded at the destination of the test well device 375. In one example, the measurement wells 515 may be separated from the extractant wells 505, test wells 405, and filters 510, to reduce the weight and size of the test well device 375 prior to transport. In addition to filtering, the test well device 375 may also be affixed to a centrifuge for separating the contents of the test wells, for example to separate the soil from the filtrate or the supernatant.

To enhance measurement accuracy, test wells 405 in the test well device 375 may come preloaded one or more standard solutions that contain known amounts of phosphorus, phosphates, potassium, zinc, sulfur, sulphate, manganese, nitrate, nitrite or other chemical elements. These standard solutions can be used to provide reference measurements.

### Pricing

Automated processing and measuring field samples in a liquid form greatly reduces the time, labor, and shipping cost associated with agrometric measurements. The savings are pronounced in comparison to the cost associated with the traditional dry and grind method of field sample testing. Further, the use a test well device containing soil-water subsamples streamlines and parallelizes the process of handling and measuring many field samples. The time and cost savings associated with using the described device and processes allow a sample owner to greatly increase the number of field samples they measure, as well as increase the frequency with which they test their fields. Currently, it is commonplace to measure a field sparsely in terms of the density of field samples taken per unit area, typically on the order of once per 10000m² (2.5 acres) or less. Further, it is commonplace to test fields once per two to four years, at most. By fitting a number of test samples into each test well device 375, the cost of shipping test samples is reduced, making it economically feasible to ship test samples long distance at low cost. Low cost shipping of test samples allows for consolidation of sample testing, such that a single large testing laboratory could receive test samples from thousands of miles away, thereby reducing overhead costs that would arise from maintaining multiple, local testing centers.

Using the process described herein, it is cost and labor effective to measure a field more densely and more frequently than was previously possible. For an organization that provides a service of testing field samples, it is possible to price and charge for field sample testing on a per 4000 m² (per-acre) basis, largely independent of the density of field sample measurements. Thus, even if the field sample measurements are taken at a density of, for example, 10 or 20 samples per 4000 m² (per acre), the cost of measuring the samples does not rise appreciably for the organization providing the testing compared to existing measurement techniques.

Alternatively, an organization that provides a service of testing field samples may adjust their price for field sample testing to include the costs of consumables used in field testing and remote laboratory testing. For example, the test well device 375 may be a disposable product which $1 (for example) per test well 405, or $24 per test well device 375. The cost may be higher if a reusable test well device 375 is used. The cost of shipping the test well device 375 between the location where the test well device 375 is prepared and the remote laboratory may also be included in the cost of consumables.

Other consumables involved in the process of testing may also be included in the consumables cost, includes packaging for the test well, seals or filters 510 isolating extractant wells 505 from test wells 405 and measurement wells 515. Further, if a device 300 used to process field samples and add subsamples to the test well device, this device 300 may have removable or disposable parts which can be purchased by the soil owner. For example, pH buffer solution 355, extractants themselves 350 or replacement probes 320, 325, or 330 may all be included as consumable costs that may be factored into the pricing scheme for testing.

Thus, it is possible to price and charge for field sample testing based on the size of the area sampled, largely independent of the density of field sample measurements, and also incorporating any consumable costs. Even factoring in high density field sampling and the costs of consumables, the cost of measuring the samples does not rise appreciably for the organization providing the testing.

Pricing for field sample testing can also be provided on the basis of the amount of time taken to process and/or measure a given number of field samples. As measurements can be performed in the field where the sample was taken, or at a field office located in close proximity to the field where the sample was taken, it is possible to provide field sample analysis results in a shorter time frame that was previously possible. A sample testing organization may provide pricing and charge sample owners for an amount of time spent collecting and measuring, independent of the number of field samples processed. For example, the sample owner may pay hourly for field sample testing, independent of the number of field samples tested.

Providing a sample owner with higher density field sample testing tied with a location where a sample was taken, allows a sample owner to make more finely tuned decisions about how much fertilizer, pesticide, or other growing materials to place on a field. This can save money that would have otherwise been wasted on over-fertilization, for example. Field sample labeling and tracking provide additional information which increase the benefit provided to the sample owner.

### Additional Considerations

A process and device for homogenizing and measuring soil-water samples removes the need to dry and grind soil before measurement. Measuring field samples in a soil-water suspension allows for faster turn-around time between when a soil subsample is received for measurement and when a measurement result can be provided, as compared to the turn-around time for dry and grind type measurement processes. For example, using soil-water samples a chemical analysis of the soil can be provided within minutes of receiving a field sample, versus the 4-48 hour wait time usually required for dry and grind processes.

A faster measurement turn-around time allows some time-sensitive soil measurements to be performed near the field where the sample is taken, as opposed to having to wait until the sample reaches a remote laboratory. This is beneficial for measurements where the property of the soil to be measured degrades the longer the soil sits before it is measured. For example, nitrate-nitrogen concentration in soil, which degrades if the sample is dried and transported, may be measured in a soil-water format near the field in a short period of time.

Measuring field samples in a soil-water suspension also improves the quality of soil measurements. The quality of a soil measurement is judged in part based on whether the soil subsample measurement result is representative of the soil sample as a whole. Increasing the homogeneity of the soil sample ensures that the soil subsample is representative of the soil it is taken from. It is easier to achieve a higher degree of homogeneity in soil-water samples than with dry samples. A sample has a high homogeneity if the particulates that make up the sample are relatively small, as large particulate clumps contribute less to the measurement of a sample, thus resulting in a biased measurement. With soil-water samples, the presence of water and the ease of mixing liquid suspensions make the process of breaking up soil clumps produce better subsamples. Soil-water suspensions are also able to obtain a finer granularity than dried, ground samples. Consequently, soil-water sample measurements are more representative of the soil as a whole than their dry and grind equivalents. For example, measuring potassium in a soil-water format provides a measurement that correlates with corn and soy nutrient uptake response and yield response better than traditional dry and grind methods.

Further, drying and grinding is known to produce inconsistent measurements of soil nutrients. Drying, particularly, is known to affect the mineralogy of field samples, resulting inaccurate measurements of soil nutrients. Wet measurement, in contrast, avoids problems associated with drying, thereby improving the precision of soil nutrient measurements.

The addition of labels to the containers of soil-water samples allows for improved field sample tracking. This removes the need for additional labor associated with cataloging field samples as they arrive at a laboratory for analysis from the field. Further, labeling and tracking allows measurement results to be communicated to farmers or their representative agents as soon as they are available. This information can aid in the scheduling, staffing requirements, and speed of subsequent measurements, thus providing better performance, process tracking and reliability to the sample owner.

## Claims

1. A method of measuring an attribute of soil taken from a location in a field, the method comprising:
receiving a soil subsample originating from soil taken from the location in the field;
measuring (135) a moisture content of the soil subsample;
choosing a dilution ratio based on the attribute of the soil to be measured;
based on the measured moisture content of the soil subsample, mixing (140, 145) water with the soil subsample until the chosen dilution ratio is achieved, thereby creating a soil-water mixture;
selecting (150) a soil-water subsample from the soil-water mixture, the soil-water subsample representative of the soil-water mixture;
analyzing the soil-water subsample; and
determining (170) the attribute of the soil, based on the analysis of the soil-water subsample.

2. The method of claim 1 comprising:
obtaining a field sample at a first location;
obtaining (110) characterization data related to the field sample; and
sending the field sample to a second location where the attribute of the soil is determined based on the field sample.

3. The method of claim 2 wherein the characterization data comprises at least one of: a location where the field sample was obtained, an entity responsible for the field sample, a time the sample was obtained, a date the sample was obtained, and an identification for the field sample.

4. The method of claim 1 comprising:
receiving (105) a field sample; and
selecting (130) the soil subsample from a field sample, the soil subsample representative of the field sample.

5. The method of claim 4 comprising performing filtering (120) on the field sample, the filtering comprising passing the field sample through a screen.

6. The method of any preceding claim comprising adding (155) an extractant to the soil-water subsample.

7. The method of any preceding claim comprising performing filtering (165) on the soil-water subsample prior to measurement, the filtering comprising passing the soil-water subsample through a paper filter or centrifuging the soil-water subsample.

8. The method of any preceding claim wherein the soil subsample has a weight between 100 grams and 1 kilogram, inclusive.

9. The method of any preceding claim wherein the dilution ratio is between one part water to two parts soil subsample, to five parts water to one part soil subsample, inclusive.

10. The method of any preceding claim comprising:
selecting (245) a second soil-water subsample from the soil-water mixture, and transferring (250) the second soil-water subsample into a test well device; and
sending (260) the test well device to a remote location for determining a second attribute the soil at the location, based on analysis of the second soil-water subsample.

## Patentansprüche

1. Verfahren zum Messen einer Eigenschaft von Boden, welcher von einer Stelle in einem Feld entnommen wurde, wobei das Verfahren umfasst:
Erhalten einer Bodenteilprobe, die von Boden stammt, der von der Stelle im Feld entnommen wurde;
Messen (135) eines Feuchtigkeitsgehalts der Bodenteilprobe;
Auswählen eines Verdünnungsverhältnisses basierend auf der Eigenschaft des zu messenden Bodens;
Mischen (140, 145) von Wasser mit der Bodenteilprobe, basierend auf dem gemessenen Feuchtigkeitsgehalt der Bodenteilprobe, bis das gewählte Verdünnungsverhältnis erreicht ist, wodurch eine Boden-Wasser-Mischung erzeugt wird;
Auswählen (150) einer Boden-Wasser-Teilprobe aus der Boden-Wasser-Mischung, wobei die Boden-Wasser-Teilprobe für die Boden-Wasser-Mischung repräsentativ ist;
Analysieren der Boden-Wasser-Teilprobe; und Bestimmen (170) der Eigenschaft des Bodens, basierend auf der Analyse der Boden-Wasser-Teilprobe.

2. Verfahren nach Anspruch 1, umfassend:
Gewinnen einer Feldprobe an einem ersten Ort;
Gewinnen (110) von Charakterisierungsdaten bezüglich der Feldprobe;
und
Senden der Feldprobe an einen zweiten Ort, an dem die Eigenschaft des Bodens basierend auf der Feldprobe bestimmt wird.

3. Verfahren nach Anspruch 2, worin die Charakterisierungsdaten mindestens eine umfassen aus: einen Ort, an dem die Feldprobe gewonnen wurde, eine für die Feldprobe verantwortliche Stelle, eine Zeit, zu der die Probe gewonnen wurde, ein Datum, an dem die Probe gewonnen wurde, und eine Identifikation für die Feldprobe.

4. Verfahren nach Anspruch 1, umfassend:
Erhalten (105) einer Feldprobe; und
Auswählen (130) der Bodenteilprobe aus einer Feldprobe, wobei die Bodenteilprobe die Feldprobe darstellt.

5. Verfahren nach Anspruch 4, umfassend das Durchführen einer Siebung (120) der Feldprobe, wobei die Siebung das Führen der Feldprobe durch ein Sieb umfasst.

6. Verfahren nach einem der vorhergehenden Ansprüche, umfassend das Zugeben (155) eines Extraktionsmittels zu der Boden-Wasser-Teilprobe.

7. Verfahren nach einem vorhergehenden Anspruch, umfassend das Durchführen einer Filtrierung (165) der Boden-Wasser-Teilprobe vor der Messung, wobei die Filtrierung das Durchleiten der Boden-Wasser-Teilprobe durch einen Papierfilter oder das Zentrifugieren der Boden-Wasser-Teilprobe umfasst.

8. Verfahren nach einem der vorhergehenden Ansprüche, worin die Bodenteilprobe inklusiv ein Gewicht zwischen 100 Gramm und 1 Kilogramm einschließlich.

9. Verfahren nach einem der vorhergehenden Ansprüche, worin das Verdünnungsverhältnis zwischen einem Teil Wasser zu zwei Teilen Bodenteilprobe bis fünf Teile Wasser zu einem Teil Bodenteilprobe, inklusiv, beträgt.

10. Verfahren nach einem vorhergehenden Anspruch, umfassend:
Auswählen (245) einer zweiten Boden-Wasser-Teilprobe aus dem Boden-Wasser-Gemisch und Überführen (250) der zweiten Boden-Wasser-Teilprobe in eine Testbehältnisvorrichtung; und
Senden (260) der Testbehältnisvorrichtung an einen entfernten Ort zum Bestimmen einer zweiten Eigenschaft des Bodens an dem Ort, basierend auf der Analyse der zweiten Boden-Wasser-Teilprobe.

## Revendications

1. Procédé de mesure d'un attribut de sol prélevé d'un emplacement dans un champ, le procédé comprenant les étapes consistant à :
recevoir un sous-échantillon de sol provenant d'un sol prélevé de l'emplacement dans le champ ;
mesurer (135) la teneur en humidité du sous-échantillon de sol ;
choisir un rapport de dilution sur la base de l'attribut du sol à mesurer ;
sur la base de la teneur en humidité mesurée du sous-échantillon de sol, à mélanger (140, 145) de l'eau avec le sous-échantillon de sol jusqu'à ce que le rapport de dilution choisi soit obtenu, créant ainsi un mélange sol-eau ;
sélectionner (150) un sous-échantillon de sol-eau du mélange sol-eau, le sous-échantillon de sol-eau représentant le mélange sol-eau ;
analyser le sous-échantillon de sol-eau ; et
déterminer (170) l'attribut du sol, sur la base de l'analyse du sous-échantillon de sol-eau.

2. Procédé de la revendication 1 comprenant les étapes consistant à :
obtenir un échantillon de champ à un premier emplacement ;
obtenir (110) des données de caractérisation relatives à l'échantillon de champ ; et
envoyer l'échantillon de champ à un deuxième emplacement où l'attribut du sol est déterminé sur la base de l'échantillon de champ.

3. Procédé de la revendication 2, dans lequel les données de caractérisation comprennent au moins l'un(e) parmi : un emplacement où l'échantillon de champ a été obtenu, une entité chargée de l'échantillon de champ, une heure à laquelle l'échantillon a été obtenu, une date à laquelle l'échantillon a été obtenu, et une identification de l'échantillon de champ.

4. Procédé de la revendication 1, comprenant les étapes consistant à :
recevoir (105) un échantillon de champ ; et
sélectionner (130) le sous-échantillon de sol d'un échantillon de champ, le sous-échantillon de sol représentant l'échantillon de champ.

5. Procédé de la revendication 4, comprenant l'étape consistant à effectuer une filtration (120) sur l'échantillon de champ, la filtration comprenant le passage de l'échantillon de champ à travers un tamis.

6. Procédé de l'une des revendications précédentes, comprenant l'étape consistant à ajouter (155) un agent d'extraction au sous-échantillon de sol-eau.

7. Procédé de l'une des revendications précédentes, comprenant l'étape consistant à effectuer une filtration (165) sur le sous-échantillon de sol-eau avant la mesure, la filtration comprenant le passage du sous-échantillon de sol-eau à travers un filtre en papier ou la centrifugation du sous-échantillon de sol-eau.

8. Procédé de l'une des revendications précédentes, dans lequel le sous-échantillon de sol a un poids compris entre 100 grammes et 1 kilogramme, inclus.

9. Procédé de l'une des revendications précédentes, dans lequel le rapport de dilution est compris entre une partie d'eau sur deux parties de sous-échantillon de sol et cinq parties d'eau sur une partie de sous-échantillon de sol, inclus.

10. Procédé de l'une des revendications précédentes, comprenant les étapes consistant à :
sélectionner (245) un deuxième sous-échantillon de sol-eau du mélange sol-eau, et à transférer (250) le deuxième sous-échantillon de sol-eau dans un dispositif de puits d'essai ; et
envoyer (260) le dispositif de puits d'essai à un emplacement distant pour déterminer un deuxième attribut du sol à l'emplacement, sur la base de l'analyse du deuxième sous-échantillon de sol-eau.
